(19) Europäisches Patentamt
European Patent Office
Office européen des brevets

(11) Veröffentlichungsnummer : **0 007 407**
**B1**

(12) # EUROPÄISCHE PATENTSCHRIFT

(45) Veröffentlichungstag der Patentschrift : **17.06.81**

(51) Int. Cl.³ : **G 01 N 33/52**, C 07 D327/04, G 01 N 33/68

(21) Anmeldenummer : **79101828.6**

(22) Anmeldetag : **08.06.79**

(54) **Diagnostisches Mittel zum Nachweis von Leukozyten in Körperflüssigkeiten sowie als Chromogene hierfür geeignete Sulfonphthalein-Ester, Verfahren zu deren Herstellung sowie deren Verwendung zur Herstellung diagnostischer Mittel zum Nachweis von Leukozyten in Körperflüssigkeiten.**

(30) Priorität : **20.06.78 DE 2826965**

(43) Veröffentlichungstag der Anmeldung : **06.02.80 (Patentblatt 80/03)**

(45) Bekanntmachung des Hinweises auf die Patenterteilung : **17.06.81 Patentblatt 81/24**

(84) Benannte Vertragsstaaten : **AT BE CH DE FR GB IT LU NL SE**

(56) Entgegenhaltungen :
**GB - A - 1 128 371**
**US - A - 3 087 794**
**US - A - 3 872 046**
**US - A - 4 022 667**

(73) Patentinhaber : **BOEHRINGER MANNHEIM GMBH**
**Sandhofer Strasse 112-132 Postfach 31 01 20**
**D-6800 Mannheim 31-Waldhof (DE)**

(72) Erfinder : **Berger, Dieter, Dr.**
**Bensheimer-Strasse 45**
**D-6806 Viernheim (DE)**
Erfinder : **Braun, Franz, Dr.**
**Ringstrasse 14**
**D-6149 Rimbach Odw (DE)**
Erfinder : **Frey, Günter**
**Heinigstrasse 45**
**D-6700 Ludwigshafen (DE)**
Erfinder : **Güthlein, Werner, Dr.**
**Im Sennteich 31**
**D-6800 Mannheim-Neckarau (DE)**
Erfinder : **Werner, Wolfgang, Dr.**
**Meissener Weg 39**
**D-6800 Mannheim-42 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

# Diagnostisches Mittel zum Nachweis von Leukozyten in Körperflüssigkeiten sowie als Chromogene hierfür geeignete Sulfonphthalein-Ester, Verfahren zu deren Herstellung sowie deren Verwendung zur Herstellung diagnostischer Mittel zum Nachweis von Leukozyten in Körperflüssigkeiten

Der Nachweis von Leukozyten in Körperflüssigkeiten, insbesondere im Urin, nimmt eine hervorragende Stelle in der Diagnostik der Erkrankungen der Nieren und des Urogenitaltraktes ein.

Bisher wird dieser Nachweis durch mühsames Auszählen der Leukozyten im nicht zentrifugierten Harn oder im Harnsediment geführt. Beiden Methoden ist naturgemäß gemeinsam, daß nur intakte Leukozyten erfaßt werden. Andererseits ist bekannt, daß die Geschwindigkeit der Leukozyten-Lyse je nach Harnmilieu enormen Schwankungen unterworfen ist, so ist z.B. in stark alkalischen Harnen mit einer Leukozyten-Halbwertszeit von nur 60 Minuten zu rechnen. Zu niedrige Leukozytenzahlen bzw. bei längeren Harnstandzeiten sogar falschnegative Befunde sind die Folge.

Vom Lyse-Fehler abgesehen, liefert die quantitative mikroskopische Bestimmung der Leukozyten im nicht zentrifugierten, homogenisierten Harn in der Zählkammer recht zuverläßige Werte. In der Praxis wird diese Methode jedoch nur selten angewandt, da sie mühevoll, ermüdend und zeitraubend ist und den Einsatz geschulten Personals bedingt.

Die überwiegende Mehrzahl der Leukozytenbestimmungen im Harn werden in der medizinischen Praxis nach der sogenannten Gesichtsfeldmethode im Harnsediment durchgeführt. Hierzu muß zunächst das Untersuchungsgut (Sediment) durch Zentrifugieren gewonnen werden. Dabei werden jedoch auch andere Bestandteile des Harnes angereichert, die - wie z.B. Epithelzellen und Salze - die mikroskopische Auszählung der Leukozyten beträchtlich erschweren können. Schwankender Sedimentgehalt, Inhomogenitäten des Sedimentes sowie womöglich unterschiedliche mikroskopische Vergrößerungen oder unterschiedliche optische Ausstattung der Mikroskope führen dazu, daß die hier übliche « quantitative » Aussage über die Anzahl der Leukozyten pro mikroskopischem Gesichtsfeld mit Fehlern von mehreren hundert Prozent behaftet sein kann.

Aufgabe der vorliegenden Erfindung war es daher, ein diagnostisches Mittel bereitzustellen, mit dem die Leukozyten in Körperflüssigkeiten auf einfache und leicht zu handhabende Weise sowie möglichst schnell und vollständig nachgewiesen werden können.

Als Nachweisprinzip für einen solchen Leukozytentest bietet sich eine enzymatische Reaktion an, da die Leukozyten ein breitgefächertes Enzymspektrum besitzen.

In dem US-Patent 3 087 794 ist bereits ein Leukozytennachweis beschrieben und beansprucht, der über die in den granulozytären Leukozyten vorhandene peroxidatische Aktivität geführt wird. Ein saugfähiger Träger, der mit Wasserstoffperoxid und einem organischen Indikator, beispielsweise o-Tolidin, imprägniert ist, zeigt die Anwesenheit von Leukozyten durch die Bildung eines farbigen Oxydationsproduktes an. Ein solcher Test besitzt jedoch entscheidende Nachteile. Einerseits besitzen peroxidatische Reaktionen ganz allgemein gegenüber reduzierenden Substanzen im Harn, wie z.B. gegenüber Ascorbinsäure, eine erhebliche Störanfälligkeit. Andererseits finden sich in mehreren Literaturstellen (siehe z.B. L. Mettler, Med. Welt *23,* 399 (1972)) Hinweise auf die Instabilität der Leukozytenperoxidase im Harnmilieu, die zu falschnegativen Befunden Anlaß gibt.

Seit einigen Jahren haben in der histo- und zytochemischen Ensymologie kolorimetrische Nachweismethoden, die auf der esterolytischen Aktivität der in den zu bestimmenden Systemen vorhandener Enzyme beruhen, ihren festen Platz. (vgl. z.B. A.G.E. Pearse, Histochemistry, Theoretical and Applied). Im Prinzip werden dabei farblose oder schwach gefärbte Ester eingesetzt, die durch die enzymatische Spaltung zumeist in eine farblose Säure- und eine ebenfalls farblose Alkohol-(Phenol)-Komponente zerfallen. Letztere wird dann in einer der enzymatischen Verseifung folgenden Reaktion zu farbigen Produkten umgesetzt (z.B. Kupplung mit Diazoniumsalzen oder oxidative Reaktionen).

So beschreiben beispielsweise F. Schmalzl und H. Braunsteiner, in Klin. Wschr. *46,* 642 (1968) einen spezifischen zytochemischen Leukozytenesterase-Nachweis mit Naphthol-AS-D-chlor-acetat als Substrat und einem Diazoniumsalz zur Bildung der farbigen Azo-Verbindung.

Für ein diagnostisches Mittel zum schnellen und einfachen Nachweis von Leukozyten in Körperflüssigkeiten, wie z.B. im Harn, erweisen sich Zwei-Komponenten-Systeme dieser Art als nicht geeignet, da bekanntlich viele im Harn vorkommende Verbindungen, wie Urobilinogen, Stercobilinogen, Bilirubin u.a., mit Diazoniumsalzen reagieren. Darüberhinaus ist dieser Nachweis viel zu unempfindlich. Beispielsweise zeigen Proben mit 5 000 Leukozyten/µl keine Reaktion.

Überraschenderweise wurde nun gefunden, daß man stabile und schnell anzeigende diagnostische Mittel, mit denen Leukozyten gut in Körperflüssigkeiten nachzuweisen sind, erhält, wenn als Substrate zum Nachweis der in den neutrophilen Leukozyten-Granulozyten vorkommenden Esterasen Sulfonphthaleinester verwendet werden.

Gegenstand der vorliegenden Erfindung ist daher ein diagnostisches Mittel zum Nachweis von Leukozyten in Körperflüssigkeiten, bestehend aus einem saugfähigen, Träger, der mit einem Chromogen und üblichen Zusatzstoffen imprägniert ist, dadurch gekennzeichnet, daß als Substrat zum Nachweis für die in den Leukozyten vorhandenen Esterasen Sulfonphthaleinester der allgemeinen Formel I

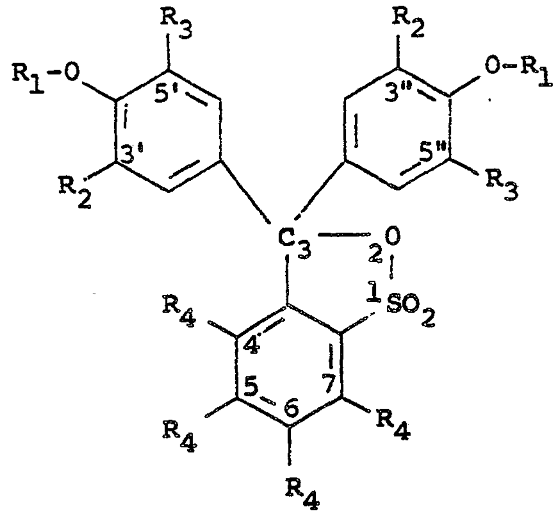

(I),

in der

$R_1$ einen gegebenenfalls durch Halogen oder eine niedere Alkoxygruppe substituierten Carbonsäurerest, einen mit einer in der Peptidchemie üblichen Stickstoffschutzgruppe versehenen Aminosäure- oder Peptidrest,

$R_2$ ein Halogenatom oder eine niedere Alkylgruppe,

$R_3$ und $R_4$, die gleich oder verschieden sein können, jeweils ein Wasserstoff- oder ein Halogenatom bedeuten,

eingesetzt werden.

Ein weiterer Gegenstand der vorliegenden Erfindung ist die Verwendung der Sulfonphthaleinester der allgemeinen Formel I zur Herstellung von diagnostischen Mitteln zum Nachweis von Leukozyten in Körperflüssigkeiten.

Die Sulfonphthaleinester der allgemeinen Formel I sind zum größten Teil neue Verbindungen. Literaturbekannt sind lediglich das Di-acetyl-3',5',3",5"-tetrabrom-phenolsulfonphthalein (= Bromphenolblau-di-acetat) und das Di-benzoyl-3',5',3",5"-tetrabrom-phenolsulfonphthalein (= Bromphenolblau-dibenzoat) (W. R. Orndorff, F. W. Sherwood, J. Amer. Chem. Soc. *45*, 486 (1923)).

Gegenstand der vorliegenden Erfindung sind daher ferner die neuen Sulfonphthaleinester der allgemeinen Formel (I')

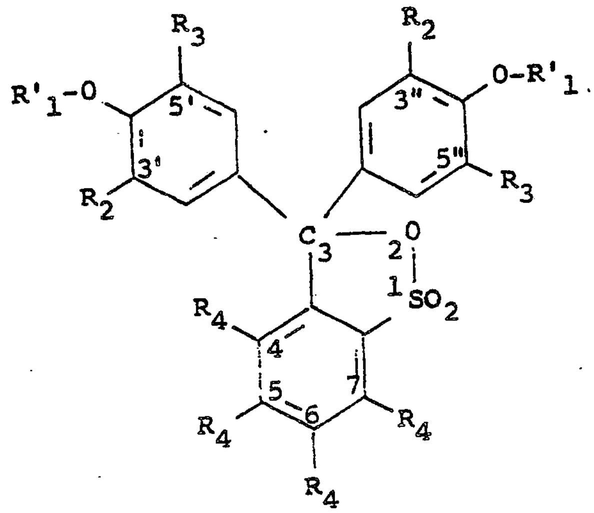

(I'),

in der

$R_2$, $R_3$ und $R_4$ die oben angegebene Bedeutung haben und $R_1'$ dieselbe Bedeutung wie $R_1$ hat, jedoch für den Fall, daß $R_2$ und $R_3$ ein Bromatom und $R_4$ ein Wasserstoffatom vorstellen, nicht Acetyl oder Benzoyl bedeutet,

sowie Verfahren zu deren Herstellung.

Die Herstellung der neuen Sulfonphthaleinester der allgemeinen Formel I' kann nach an sich bekannten Methoden erfolgen. Vorzugsweise werden in an sich bekannter Weise die entsprechenden, bekannten Sulfonphthaleine der allgemeinen Formel II

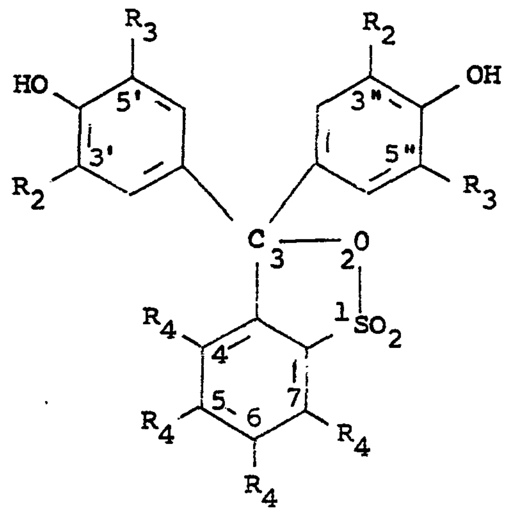

(II),

in der

$R_2$, $R_3$ und $R_4$ die oben angegebene Bedeutung haben,

mit Säuren der allgemeinen Formel III

$$HO-R_1' \quad (III),$$

in der

$R_1'$ die oben angegebene Bedeutung hat,

beziehungsweise mit geeigneten reaktiven Derivaten davon umgesetzt.

Als reaktive Derivate werden bei der Herstellung der Carbonsäureester insbesondere die entsprechenden Carbonsäureanhydride bzw. Carbonsäurechloride eingesetzt, gegebenenfalls unter Zusatz tert. Amine. Zur Herstellung der Aminosäure- und Peptid-Ester werden die in der Peptidchemie üblichen Synthesemethoden, wie z.B. die Misch-Anhydrid- und die Säurechlorid-Methode, angewendet.

Unter Halogen in der Definition der Substituenten $R_1$, $R_1'$, $R_2$, $R_3$ und $R_4$ ist Fluor, Chlor und Brom, vorzugsweise Chlor und Brom zu verstehen.

Die « niedere Alkoxygruppe » in der Definition des Substituenten $R_1$ bzw. $R_1'$ sowie die « niedere Alkylgruppe » des Substituenten $R_2$ enthalten 1 bis 5, vorzugsweise 1 bis 3 Kohlenstoffatome, wobei die Methoxy- bzw. Methylgruppe ganz besonders bevorzugt sind.

Als Carbonsäurereste des Substituenten $R_1$ bzw. $R_1'$ kommen die Reste aliphatischer Carbonsäuren mit 1 bis 7, vorzugsweise 1 bis 4 Kohlenstoffatomen, oder auch aromatischer Carbonsäuren, wie beispielsweise der Benzoe- oder Naphthoesäuren, in Frage. Besonders bevorzugt sind Acetyl-, Propionyl- und Benzoyl- reste.

Als Aminosäurereste des Substituenten $R_1$ bzw. $R_1'$ sind die Reste der natürlichen L-α-Aminosäuren, insbesondere von L-Alanin, L-Phenylalanin, L-Lysin, L-Tyrosin und L-Arginin bevorzugt, die durch eine Nitrogruppe substituiert sein können und deren eventuell vorhandene Hydroxygruppe gegebenenfalls eine übliche Sauerstoffschutzgruppe, beispielsweise eine Acetylgruppe, trägt.

Unter einem Peptidrest in der Definition des Substituenten $R_1$ bzw. $R_1'$ sind Di-, Tri- und Tetrapeptide, vorzugsweise Dipeptide zu verstehen, wobei als Aminosäure-Komponente vorzugsweise die oben erwähnten Aminosäuren Verwendung finden.

Die erfindungsgemäß als Chromogene verwendeten Sulfonphthalein-Ester der allgemeinen Formel I werden in Konzentrationen von $10^{-4}$ bis $10^{-1}$ mol/Liter, vorzugsweise $10^{-3}$ bis $10^{-2}$ mol/Liter Imprägnierlösung eingesetzt.

Ein weiterer Bestandteil des diagnostischen Mittels für den Leukozytennachweis ist ein geeignetes Puffersystem. Hierzu kommen z.B. Phosphat-, Barbiturat-, Borat-, Tris- (hydroxymethyl)-aminomethan (Tris-), 2-Amino-2-methyl-propandiol-1,3- (Amediol-) oder Aminosäure-Puffer in Frage, wobei pH-Wert und Kapazität so gewählt werden müssen, daß sich nach dem Eintauchen des Teststreifens in die Körperflüssigkeit auf diesem ein pH-Wert von 6-10, vorzugsweise von 7-9, einstellt.

Weiterhin ist es vorteilhaft, bei der Herstellung der erfindungsgemäßen diagnostischen Mittel zum Nachweis von Leukozyten in Körperflüssigkeiten zusätzlich Tenside zu verwenden, da hierdurch kürzere Reaktionszeiten erreicht werden können.

Vorzugsweise werden kationenaktive Netzmittel, wie z.B. quaternäre Pyridiniumsalze in Konzentrationen von 0.05-2 %, vorzugsweise 0.1-0.5 %, eingesetzt.

Zur Herstellung des erfindungsgemäßen diagnostischen Mittels werden vorzugsweise saugfähige Träger, wie z.B. Filterpapier, Cellulose oder Kunstfaservliese, mit Lösungen der erforderlichen, üblicherweise zur Herstellung von Teststreifen verwendete Reagenzien (Substrat, Puffer, gegebenenfalls Tenside oder auch Verdickungsmittel, wie z.B. Polyvinylpyrrolidon, Carboxymethylcellulose und Stärke,

Stabilisierungsmittel, beispielsweise Aminosäuren, Hintergrundfärbemittel, beispielsweise Tartrazin, usw.) in leichtflüchtigen Lösungsmitteln, wie z.B. Wasser, Methanol, Ethanol oder Aceton, imprägniert. Dies geschieht zweckmäßig in zwei getrennten Schritten : Zunächst wird mit einer wäßrigen Lösung imprägniert, die den Puffer enthält. Danach wird mit einer Lösung der Esterase-Substrate der allgemeinen Formel I imprägniert. In speziellen Fällen kann auch die umgekehrte Imprägnierfolge angewandt werden. Die fertigen Testpapiere können als solche verwendet werden oder in an sich bekannter Weise an Griffen angeklebt oder vorzugsweise zwischen Kunststoffen und feinmaschigen Netzwerken gemäß DBP 2 118 455 eingesiegelt werden.

Man erhält diagnostische Mittel, die nach Eintauchen in die zu untersuchende Körperflüssigkeit rasch und in einfach zu handhabender Weise die Anwesenheit von Leukozyten über eine Farbreaktion anzeigen. Da die Aktivität der in den neutrophilen Leukozyten-Granulozyten vorkommenden Esterasen auch nach der Lyse der Leukozyten voll erhalten bleibt, werden mit dem erfindungsgemäßen diagnostischen Mittel sowohl intakte als auch lysierte Leukozyten erfaßt. Ein Lysefehler tritt folglich nicht auf.

## Beispiel 1

Filterpapier (z.B. Schleicher + Schüll 23 SL) wird nacheinander mit den folgenden Lösungen imprägniert und dann bei 60 °C getrocknet :

### Lösung 1

| | |
|---|---|
| Tris-(hydroxymethyl)-aminomethan | 0,61 g |
| Salzsäure, 0.1 N | ca. 5 ml |
| Wasser, dest., ad. | 100 ml |

Die Lösung wird mit 0.1 N Salzsäure auf einen PH-Wert von 9.0 eingestellt.

### Lösung 2

| | |
|---|---|
| Di-acetyl-4,5,6,7,3′,5′,3″,5″-octabrom-phenolsulfonphthalein (= Tetrabromphenolblau-di-acetat) | 0.107 g |
| Aceton, ad. | 100 ml |

Man erhält ein weißes Testpapier, welches sich beim Eintauchen in leukozytenhaltige Harne blau verfärbt. Es lassen sich nachweisen :

5 000 Leukozyten/µl Harn in ca. 2 Minuten,
2 000 Leukozyten/µl Harn in ca. 5 Minuten,
1 000 Leukozyten/µl Harn in ca.10 Minuten,
500 Leukozyten/µl Harn in ca.15 Minuten.

Die Empfindlichkeit des Testes liegt bei etwa 500 Leukozyten/µl.

Testpapiere mit ähnlichen Eigenschaften (Empfindlichkeiten : 300-1 000 Leukozyten/µl) erhält man, wenn man anstelle von Diacetyl-4,5,6,7,3′,5′,3″,5″-octabrom-phenolsulfonphthalein (= Tetrabromphenolblau-di-acetat) die folgenden Substrate einsetzt :

a) Di-acetyl-3′,3″-dichlor-phenolsulfonphthalein
(= Chlorphenolrot-di-acetat) ergibt beim Eintauchen in leukozytenhaltigen Harn ein rotes Reaktionsprodukt.

b) Di-acetyl-5′,5″-dibrom-o-kresolsulfonphthalein
(= Bromkresolpurpur-di-acetat) ergibt ein purpurfarbenes Reaktionsprodukt.

c) Di-acetyl-3′,5′,3″,5″-tetrabrom-phenolsulfonphthalein
(= Bromphenolblau-di-acetat) ergibt ein blaues Reaktionsprodukt.

d) Di-acetyl-3′,3″-dibrom-5′,5″-dichlor-phenolsulfonphthalein
(= Bromchlorphenolblau-di-acetat) ergibt ein blaues Reaktionsprodukt.

e) Di-acetyl-4,5,6,7-tetrabrom-3′,5′,3″,5″-tetrachlor-phenolsulfonphthalein
(= Tetrabrom-tetrachlorphenolblau-di-acetat) ergibt ein blaues Reaktionsprodukt.

f) Di-chloracetyl-3′,3″-dibrom-5′,5″-dichlor-phenolsulfonphthalein
(= Bromchlorphenolblau-di-chloracetat) ergibt ein blaues Reaktionsprodukt.

g) Di-chloracetyl-3′,5′,3″,5″-tetrabrom-phenolsulfonphthalein
(= Bromphenolblau-di-chloracetat) ergibt ein blaues Reaktionsprodukt.

h) Di-chloracetyl-4,5,6,7,3′,5′,3″,5″-octabrom-phenolsulfonphthalein
(= Tetrabromphenolblau-di-chloracetat) ergibt ein blaues Reaktionsprodukt.

i) Di-(2-methoxy-propionyl)-4,5,6,7,,3',5',3",5"-octabrom-phenolsulfonphthalein
[= Tetrabromphenolblau-di-(2-methoxy-propionat)] ergibt ein blaues Reaktionsprodukt.

j) Di-benzoyl-4,5,6,7,3',5',3",5"-octabrom-phenolsulfonphthalein
(= Tetrabromphenolblau-di-benzoat) ergibt ein blaues Reaktionsprodukt.

## Beispiel 2

Filterpapier (z.B. Schleicher + Schüll 23 SL) wird nacheinander mit den folgenden Lösungen imprägniert und dann bei 60 °C getrocknet :

### Lösung 1

| | |
|---|---|
| Di-natrium-tetraborat-decahydrat | 1.91 g |
| Salzsäure, 0.1 N | ca. 20 ml |
| Wasser, dest., ad. | 100 ml |

Die Lösung wird mit 0.1 N Salzsäure auf einen pH-Wert von 9.0 eingestellt.

### Lösung 2

| | |
|---|---|
| Di-(N-benzyloxycarbonyl-L-phenylalanyl)-3',5',3",5"-tetrabrom-phenolsulfonphthalein [= Di-(N-benzyloxycarbonyl-L-phenylalanyl)-bromphenolblau] | 0.123 g |
| Aceton, ad. | 100 ml |

Man erhält ein weißes Testpapier, das seich beim Eintauchen in leukozytenhaltige Harne nach ca. 10 Minuten blau verfärbt. Die Empfindlichkeit des Testes liegt bei etwa 1 000 Leukozyten/μl.

Testpapiere mit ähnlichen Eigenschaften (Empfindlichkeiten: 800-3 000 Leukozyten/μl) erhält man, wenn man anstelle von Di-(N-benzyloxycarbonyl-L-phenylalanyl)-3',5',3",5"-tetrabrom-phenolsulfonphthalein die folgenden Substrate einsetzt, wobei sich in allen Fällen beim Eintauchen in leukozytenhaltige Harne blaue Reaktionsprodukte ergeben:

a) Di-(N-benzyloxycarbonyl-L-alanyl)-3',5',3",5"-tetrabrom-phenolsulfonphthalein
[= Di-(N-benzyloxycarbonyl-L-alanyl)-bromphenolblau]

b) Di-(Nα,Nω-di-benzyloxycarbonyl-L-lysyl)-3',5',3",5"-tetrabrom-phenolsulphonphthalein
[= Di-Nα,Nω-di-benzyloxycarbonyl-L-lysyl-bromphenolblau]

c) Di-(N-benzyloxycarbonyl-O-acetyl-L-tyrosyl)-3',5',3",5"-tetrabrom-phenolsulfonphthalein
[= Di-(N-benzyloxycarbonyl-O-acetyl-L-tyrosyl)-bromphenolblau]

d) Di-(N-benzyloxycarbonyl-N-ethoxycarbonyl-O-acetyl-L-tyrosyl)-3',5',3",5"-tetrabrom-phenolsulphonphthalein
[= Di-(N-benzyloxycarbonyl-N-ethoxycarbonyl-O-acetyl-L-tyrosyl)-bromphenolblau]

e) Di-(Nα-benzyloxycarbonyl-Nω-nitro-L-arginyl)-3',5',3",5"-tetrabrom-phenolsulfonphthalein
[= Di-(Nα-benzyloxycarbonyl-Nω-nitro-L-arginyl)-bromphenolblau]

f) Di-(N-benzyloxycarbonyl-L-alanyl-L-alanyl)-3',5',3",5"-tetrabrom-phenolsulfonphthalein
[= Di-(N-benzyloxycarbonyl-L-alanyl-L-alanyl)-bromphenolblau]

g) Di-(N-benzyloxycarbonyl-L-alanyl)-4,5,6,7,3',5',3",5"-octabrom-phenolsulfonphthalein
[= Di-(N-benzyloxycarbonyl-L-alanyl)-tetrabromphenolblau]

## Beispiel 3

Filterpapier (z.B. Schleicher + Schüll 23 SL) wird nacheinander mit den folgenden Lösungen imprägniert und dann bei 60 °C getrocknet:

### Lösung 1

| | |
|---|---|
| Tris-(hydroxymethyl)-aminomethan | 0.61 g |
| Salzsäure, 0.1 N | ca. 5 ml |
| Laurylpyridiniumchlorid | 0.2 g |
| Wasser, dest., ad. | 100 ml |

Die Lösung wird mit 0.1 N Salzsäure auf einen pH-Wert von 9.0 eingestellt.

Lösung 2

Di-acetyl-4,5,6,7,3',5',3'',5''-octabrom-phenolsulfonphthalein (= Tetrabromphenolblau-di-acetat) 0.107 g
Aceton, ad. 100 ml

Man erhält ein weißes Testpapier, welches sich beim Eintauchen in leukozytenhaltige Harne nach ca. 1 Minute blau verfärbt. Die Empfindlichkeit des Testes liegt bei etwa 500 Leukozyten/µl.

Auch mit den anderen Substraten der Beispiele 1 und 2 werden mit Netzmitteln, wie beispielsweise dem oben verwendeten Lauryl-pyridiniumchlorid, Testpapiere erhalten, die gegenüber analogen Testpapieren ohne Netzmittel auf die Hälfte verkürzte Reaktionszeiten aufweisen.

## Beispiel 4

Di-acetyl-4,5,6,7,3',5',3'',5''-octabrom-phenolsulfonphthalein (= Tetrabromphenolblau-di-acetat)

Man löst 5.0 g (5 mmol) Tetrabromphenolblau unter Erwärmen in 54 g = 50 ml (0.45 mol) frisch destilliertem Acetanhydrid und erhitzt 3 h unter Rückfluss. Nach Einengen der Lösung im Vakuum rührt man den öligen Rückstand mit wenig Isopropanol an und kristallisiert aus Toluol um. Das so erhaltene Diacetat schmilzt bei 166-167 °C und enthält 2 mol Kristallessigsäure, die beim Trocknen über Diphosphorpentoxid bei 60 °C abgegeben werden. Man erhält 4.2 g = 77.4 % Tetrabrom-phenolblau-di-acetat, farblose Kristalle, Schmp. 174 °C (Zers.).

In analoger Weise erhält man aus den entsprechend substituierten Phenolsulfonphthaleinen die folgenden Verbindungen:

a) Di-acetyl-3',3''-dichlor-phenolsulfonphthalein
(= Chlorphenolrot-di-acetat), farblose Kristalle, Schmp. 132 °C (Zers.)

b) Di-acetyl-5',5''-dibrom-o-kresolsulfonphthalein
(= Bromkresolpurpur-di-acetat), farblose Kristalle, Schmp. 117 °C (Zers.)

c) Di-acetyl-3',3''-dibrom-5',5''-dichlor-phenolsulfonphthalein
(= Bromchlorphenolblau-di-acetat), farblose Kristalle, Schmp. 217 °C (Zers.)

d) Di-acetyl-4,5,6,7-tetrabrom-3',5',3'',5''-tetrachlor-phenolsulfonphthalein
(= Tetrabrom-tetrachlorphenolblau-di-acetat), farblose Kristalle, Schmp. 253-254 °C (Zers.)

## Beispiel 5

Di-chloracetyl-4,5,6,7,3',5',3'',5''-octabrom-phenolsulfonphthalein (= Tetrabromphenolblau-di-chloracetat)

Zu einer Lösung von 5.0 g (5 mmol) Tetrabromphenolblau in 100 ml abs. Tetrahydrofuran gibt man unter Rühren 0.81 ml (10 mmol) abs. Pyridin und tropft unter Kühlung bei 10 °C eine Lösung von 1.18 g = 0.79 ml (10.5 mmol) frisch destilliertes Chloracetylchlorid in 3 ml abs. Tetrahydrofuran zu. Nach 2 Stunden Rühren bei Raumtemperatur wird das gebildete Pyridinhydrochlorid abgesaugt und das Filtrat im Vakuum bei 50 °C eingeengt. Man erhält 6.1 g öligen Rückstand; dieser wird mit 50 ml Isopropanol aufgekocht, die unlöslichen Anteile werden abgetrennt. Nach Kühlung im Eisbad isoliert man 3.9 g = 68.7 % Tetrabromphenolblau-di-chloracetat, schwach gelbliche Kristalle, Schmp. 226-227 °C (Zers.).

In analoger Weise erhält man aus den entsprechend substituierten Phenolsulfonphthaleinen und den entsprechenden Säurechloriden die folgenden Verbindungen:

a) Di-chloracetyl-3',5',3'',5''-tetrabrom-phenolsulfonphthalein
(= Bromphenolblau-di-chloracetat), farblose Kristalle, Schmp. 206-207 °C (Zers.)

b) Di-chloracetyl-3',3''-dibrom-5',5''-dichlor-phenolsulfonphthalein
(= Bromchlorphenolblau-di-chloracetat), farblose Kristalle, Schmp. 172 °C (Zers.)

c) Di-(2-methoxy propionyl)-4,5,6,7,3',5',3'',5''-octabrom-phenolsulfonphthalein
[= Tetrabromphenolblau-di-(2-methoxy-propionat)], farblose Kristalle, Schmp. 214-216 °C (Zers.)

d) Di-benzoyl-4,5,6,7,3',5',3'',5''-octabrom-phenolsulfonphthalein
(= Tetrabromphenolblau-di-benzoat), farblose Kristalle, Schmp. 196-197 °C (Zers.)

## Beispiel 6

Di-(N-benzyloxycarbonyl-L-alanyl)-3'5',3'',5''-tetrabrom-phenolsulfonphthalein [= Di-(N-benzyloxycarbonyl-L-alanyl)-bromphenolblau]

Lösung 1

Zur Herstellung des gemischten Anhydrids werden 3.13 g (14 mmol) N-Benzyloxycarbonyl-L-alanin in 50 ml abs. Tetrahydrofuran gelöst, mit 1.93 ml (14 mmol) Triethylamin versetzt und auf − 15 °C bis − 20 °C abgekühlt. Dann werden unter Rühren 1.34 ml (14 mmol) Chlorameisensäureethylester zupipettiert und das Reaktionsgemisch unter Wasserausschluss 20-30 Minuten im Kältebad belassen.

Lösung 2

4.69 g (7 mmol) Bromphenolblau werden in 45 ml abs. Tetrahydrofuran gelöst und auf − 10 °C bis − 15 °C gekühlt.

Umsetzung

Das aus der Lösung 1 bei der Bildung des gemischten Anhydrids ausgefallene Triethylaminhydrochlorid wird rasch abgesaugt und das Filtrat zur Lösung 2 gegeben. Man fügt noch 2 ml Pyridin hinzu und rührt unter Feuchtigkeitsausschluss bei − 15 °C, wobei langsam Kohlendioxid entweicht.

Nach ca. 2 Stunden gibt man zum zweiten Mal, nach ca. 16 Stunden zum dritten Mal die gleiche Menge frisch bereitetes, abgesaugtes gemischtes Anhydrid (Lösung 1) hinzu und rührt dann noch ca. 5 Stunden bei − 15 °C weiter.

Zur Aufarbeitung wird das Reaktionsgemisch mit einigen Tropfen Wasser versetzt (zur Zersetzung von überschüssigem Anhydrid) und das Lösungsmittel im Vakuum abdestilliert. Der Rückstand wird in 100 ml Essigester aufgenommen und nacheinander mit 30 ml 1 N Zitronensäure, 20 ml Wasser, 70 ml 7%-iger Natriumhydrogencarbonat-lösung und zweimal mit 25 ml Wasser gewaschen. Nach dem Trocknen über Natriumsulfat wird die Essigesterphase im Vakuum eingedampft. Es ergeben sich 11.3 g gelbliches, klebriges Rohprodukt, das säulenchromatographisch an Kieselgel mit einem Toluol-Dioxan-Essigester-Gemisch (9:2:1) gereinigt wird.

Man erhält so 5.5 g (68 %) Di-(N-benzyloxycarbonyl-L-alanyl)-3′,5′,3″,5″-tetrabrom-phenolsulfonphthalein als farbloses, amorphes Pulver, $\alpha^{20}_D = -33.6°$ (c = 1 %, Methanol).

Laut Analyse enthält die Verbindung noch 0.39 mol Wasser, 0.19 mol Dioxan, 0.60 mol Toluol.

In analoger Weise erhält man die folgenden Verbindungen durch Umsetzung der entsprechend substituierten Phenolsulfonphthaleine mit verschiedenen Aminosäuren:

a) Di-(N-benzyloxycarbonyl-L-phenylalanyl)-3′,5′,3″,5″-tetrabrom-phenolsulfonphthalein [= Di-(N-benzyloxycarbonyl-L-phenylalanyl)-bromphenolblau], farbloses, amorphes Pulver, $\alpha^{20}_D = -33.5°$ (c = 1 %, Essigester)

b) Di-(Nα,Nω-di-benzyloxycarbonyl-L-lysyl)-3′,5′,3″,5″-tetrabrom-phenolsulfonphthalein [= Di-(Nα,Nω-di-benzyloxycarbonyl-L-lysyl)-bromphenolblau], farbloses, amorphes Pulver, $\alpha^{20}_D = -15.0°$ (c = 1 %, Essigester)

c) Di-(N-benzyloxycarbonyl-O-acetyl-L-tyrosyl)-3′,5′,3″,5″-tetrabrom-phenolsulfonphthalein [= Di-(N-benzyloxycarbonyl-O-acetyl-L-tyrosyl)-bromphenolblau], farbloses, amorphes Pulver, das 0.6 mol Essigester enthält, $\alpha^{20}_D = -35.3°$ (c = 1 %, Essigester)

Als Nebenprodukt der Synthese fällt hierbei noch an:
Di-(N-benzyloxycarbonyl-N-ethoxycarbonyl-O-acetyl-L-tyrosyl)-3′,5′,3″,5″-tetrabrom-phenolsulfonphthalein [= Di-(N-benzyloxycarbonyl-N-ethoxycarbonyl-O-acetyl-L-tyrosyl)-bromphenolblau], farbloses, amorphes Pulver, $\alpha^{20}_D = -19.5°$ (c = 1 %, Essigester)

d) Di-(N-benzyloxycarbonyl-L-alanyl)-4,5,6,7,3′,5′,3″,5″-octabrom-phenolsulfonphthalein [= Di-(N-benzyloxycarbonyl-L-alanyl)-tetrabrom-phenolblau], farbloses, amorphes Pulver, $\alpha^{20}_D = -28.7°$ (c = 1 %, Methanol)

Beispiel 7

Di-(N-benzyloxycarbonyl-L-alanyl-L-alanyl)-3′,5′,3″,5″-tetrabrom-phenolsulfonphthalein [= Di-(N-benzyloxycarbonyl-L-alanyl-L-alanyl)-bromphenolblau]

Lösung 1

Zur Herstellung des Säurechlorids nach der Einstufenmethode werden 1.18 g (4 mmol) N-Benzyloxycarbonyl-L-alanin-L-alanin in 10 ml abs. Dimethylformamid gelöst und auf − 30 °C abgekühlt. Dann werden unter Rühren und Kühlen 0.32 ml (4.4 mmol) Thionylchlorid zupipettiert und das Reaktionsgemisch unter Wasserausschluss 30 Minuten im Kältebad (– 30 °C) belassen.

Lösung 2

1.34 g (2 mmol) Bromphenolblau werden in 10 ml abs. Dimethylformamid gelöst und auf – 10 °C bis – 20 °C abgekühlt.

Umsetzung

Man gibt Lösung 2 zu Lösung 1, fügt 1 ml Pyridin hinzu und rührt 2 Stunden bei – 10 °C bis – 20 °C, darauf 2 Stunden bei Raumtemperatur. Dieselben Mengen von frisch hergestelltem Säurechlorid (Lösung 1) sowie Pyridin werden insgesamt noch dreimal zugefügt, wobei jeweils die o.a. Reaktionsbedingungen eingehalten werden.

Zur Aufarbeitung wird das Reaktionsgemisch im Vakuum zur Trockene gebracht und dann, wie in Beispiel 6 angegeben, weiterbehandelt.

Man erhält 2.67 g amorphes Rohprodukt, das säulenchromatographisch an Kieselgel mit einem Heptan-Essigsäureethylester-Gemisch (1:2) gereinigt wird.

Es ergeben sich 1.4 g (57 %) Di-(N-benzyloxycarbonyl-L-alanyl-L-alanyl)-3',5',3'',5''-tetrabrom-phenolsulfonphthalein als farbloses, amorphes Pulver, das 0.6 mol Essigsäureethylester enthält; $\alpha^{20}_D = -5.9°$ (c = 1 %, Essigester).

In analoger Weise wird die folgende Verbindung erhalten:

a) Di-(Nα-benzyloxycarbonyl-Nω-nitro-L-arginyl)-3',5',3'',5''-tetrabrom-phenolsulfonphthalein [= Di-(Nα-benzyloxycarbonyl-Nω-nitro-L-arginyl)-bromphenolblau], farbloses, amorphes Pulver, das 0.6 mol Chloroform enthält, $\alpha^{20}_D = -12.0°$ (c = 1 %, Eisessig).

**Ansprüche**

1. Diagnostisches Mittel zum Nachweis von Leukozyten in Körperflüssigkeiten, bestehend aus einem saugfähigen Träger, der mit einem Chromogen und einer geeigneten Puffersubstanz imprägniert ist, dadurch gekennzeichnet, daß als Chromogene Sulfonphthalein-Ester der allgemeinen Formel I

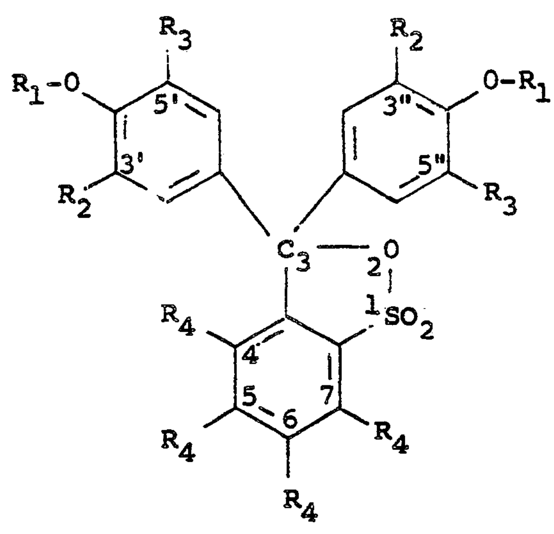

(I),

in der

$R_1$ einen gegebenenfalls durch Halogen oder eine niedere Alkoxygruppe substituierten Carbonsäurerest, einen mit einer in der Peptidchemie üblichen Stickstoffschutzgruppe versehenen Aminosäure- oder Peptidrest,

$R_2$ ein Halogenatom oder eine niedere Alkylgruppe,

$R_3$ und $R_4$, die gleich oder verschieden sein können, jeweils ein Wasserstoff- oder ein Halogenatom bedeuten, eingesetzt werden.

2. Diagnostisches Mittel gemäß Anspruch 1, dadurch gekennzeichnet, daß der Träger zusätzliche, üblicherweise verwendete Hilfsstoffe enthält.

3. Diagnostisches Mittel gemäß Anspruch 2, dadurch gekennzeichnet, daß der Träger als zusätzlichen Hilfsstoff ein Netzmittel enthält.

4. Verwendung von Sulfonphthalein-Estern der allgemeinen Formel I,

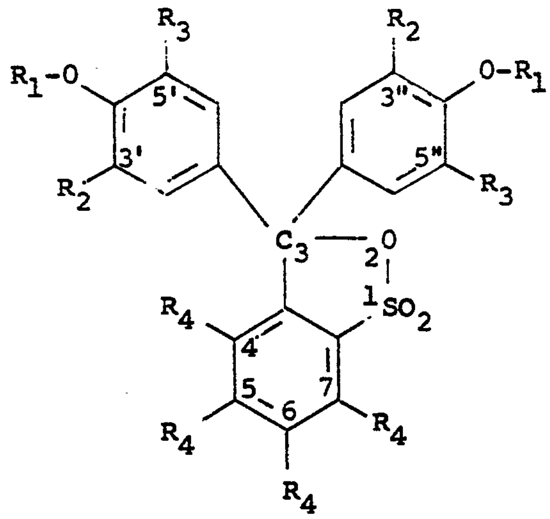

(I),

in der

$R_1$ einen gegebenenfalls durch Halogen oder eine niedere Alkoxygruppe substituierten Carbonsäurerest, einen mit einer in der Peptidchemie üblichen Stickstoffschutzgruppe versehenen Aminosäure- oder Peptidrest,

$R_2$ ein Halogenatom oder eine niedere Alkylgruppe,

$R_3$ und $R_4$, die gleich oder verschieden sein können, jeweils ein Wasserstoff- oder ein Halogenatom bedeuten, zur Herstellung diagnostischer Mittel zum Nachweis von Leukozyten.

5. Sulfonphthalein-Ester der allgemeinen Formel I'

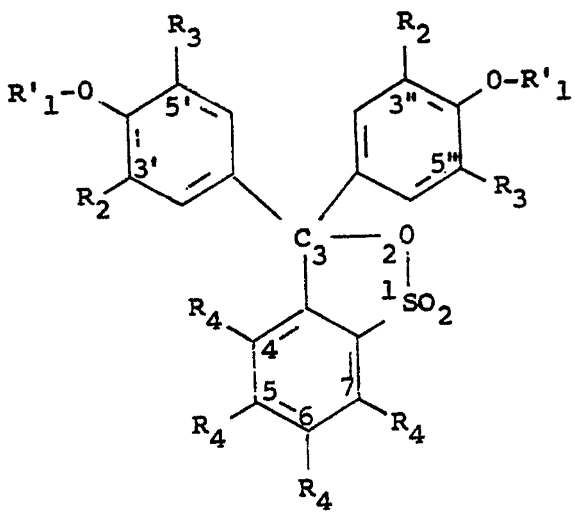

(I'),

in der

$R_1'$ einen gegebenenfalls durch Halogen oder eine niedere Alkoxygruppe substituierten Carbonsäurerest, einen mit einer in der Peptidchemie üblichen Stickstoffschutzgruppe versehenen Aminosäure- oder Peptidrest,

$R_2$ ein Halogenatom oder eine niedere Alkylgruppe,

$R_3$ und $R_4$, die gleich oder verschieden sein können, jeweils ein Wasserstoff- oder ein Halogenatom bedeuten,

wobei $R_1'$, für den Fall, daß $R_2$ und $R_3$ ein Bromatom und $R_4$ ein Wasserstoffatom vorstellen, nicht Acetyl oder Benzoyl bedeutet.

6. Verfahren zur Herstellung von Sulfonphthalein-Estern der allgemeinen Formel I'

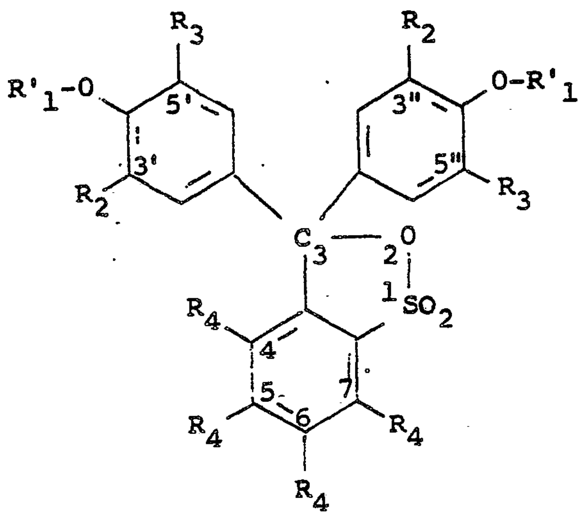

(I'),

in der

$R_1'$ einen gegebenenfalls durch Halogen oder eine niedere Alkoxygruppe substituierten Carbonsäurerest, einen mit einer in der Peptidchemie üblichen Stickstoffschutzgruppe versehenen Aminosäure- oder Peptidrest,

$R_2$ ein Halogenatom oder eine niedere Alkylgruppe,

$R_3$ und $R_4$, die gleich oder verschieden sein können, jeweils ein Wasserstoff- oder ein Halogenatom bedeuten,

wobei $R_1'$, für den Fall, daß $R_2$ und $R_3$ ein Bromatom und $R_4$ ein Wasserstoffatom vorstellen, nicht Acetyl oder Benzoyl bedeutet, dadurch gekennzeichnet, daß man in an sich bekannter Weise Verbindungen der allgemeinen Formel II

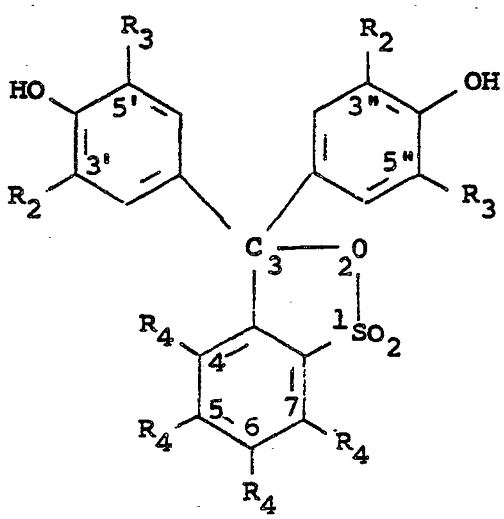

(II),

in der

$R_2$, $R_3$ und $R_4$ die oben angegebene Bedeutung haben,

mit Säuren der allgemeinen Formel III

$$HO-R_1' \quad (III)$$

in der

$R_1'$ die oben angegebene Bedeutung hat,

beziehungsweise mit geeigneten reaktiven Derivaten davon umsetzt.

**Claims**

1. Diagnostic agent for the detection of leukocytes in body fluids, consisting of an absorbent carrier which is impregnated with a chromogen and a suitable buffer substance, characterised in that, as chromogens, there are used sulphonphthalein esters of the general formula I

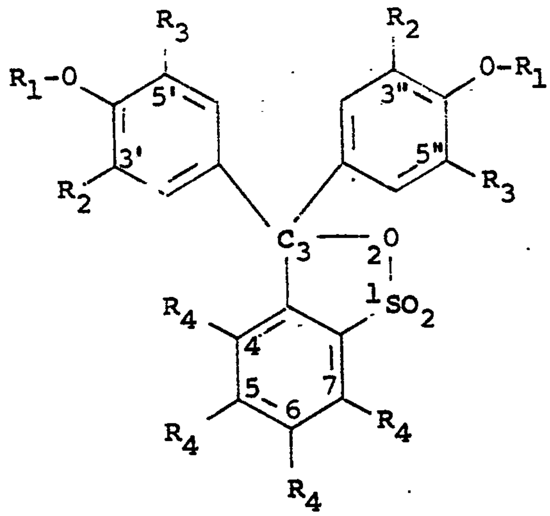

(I),

in which

$R_1$ signifies a carboxylic acid residue possibly substituted by halogen or a lower alkoxy group, an amino acid or peptide residue provided with a nitrogen protective group conventional in peptide chemistry,

$R_2$ a halogen atom or a lower alkyl group,

$R_3$ and $R_4$, which can be the same or different, each a hydrogen or a halogen atom.

2. Diagnostic agent according to claim 1, characterised in that the carrier additionally contains conventionally used adjuvants.

3. Diagnostic agent according to claim 2, characterised in that the carrier contains a wetting agent as an additional adjuvant.

4. Use of sulphonphthalein esters of the general formula I

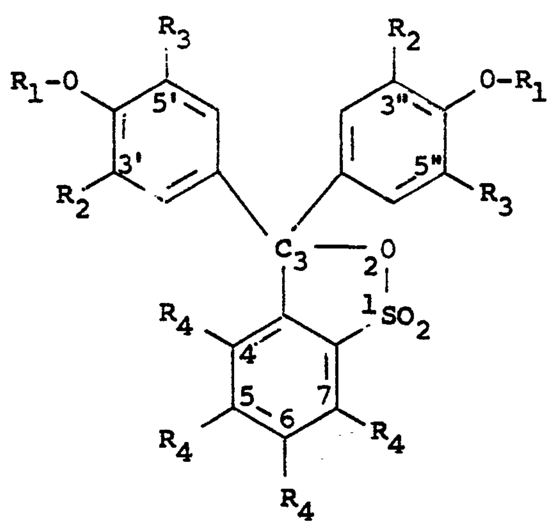

(I),

wherein

$R_1$ signifies a carboxylic acid residue possibly substituted by halogen or a lower alkoxy group, an

amino acid or peptide residue provided with a nitrogen protective group conventional in peptide chemistry and

$R_2$ a halogen atom or a lower alkyl group,

$R_3$ and $R_4$, which can be the same or different, each a hydrogen or halogen atom, for the production of diagnostic agents for the detection of leukocytes.

5. Sulphonphthalein esters of the general formula I'

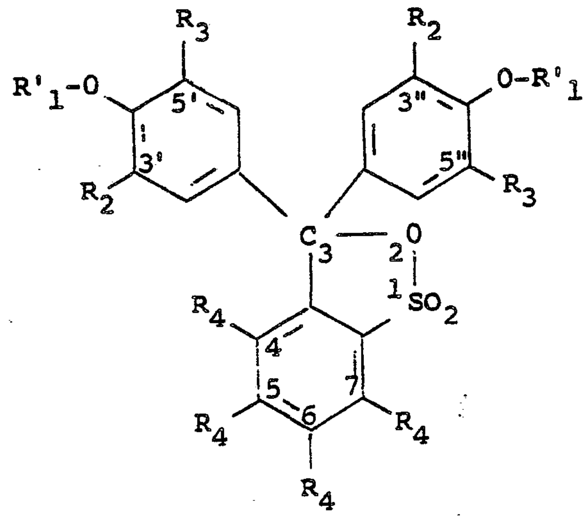

(I'),

wherein

$R'_1$ signifies a carboxylic acid residue possibly substituted by halogen or a lower alkoxy group, an amino acid or peptide residue provided with a nitrogen protective group conventional in peptide chemistry,

$R_2$ a halogen atom or a lower alkyl group,

$R_3$ and $R_4$, which can be the same of different, each a hydrogen or halogen atom, whereby, for the case in which $R_2$ and $R_3$ represents a bromine atom and $R_4$ a hydrogen atom,

$R'_1$ does not signify acetyl or benzoyl.

6. Process for the preparation of sulphonphthalein esters of the general formula I'

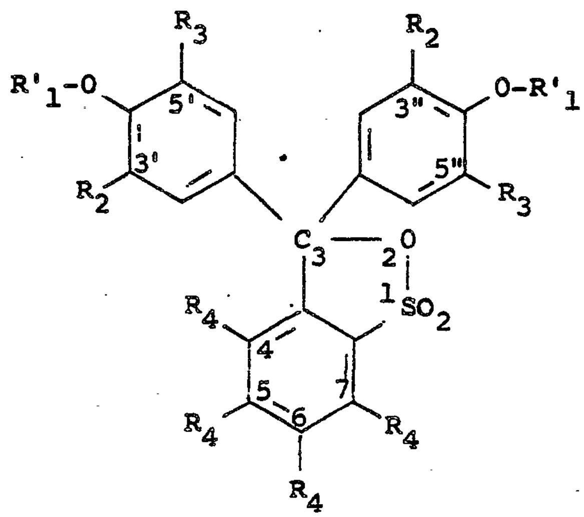

(I'),

in which

$R'_1$ is a carboxylic acid residue possibly substituted by halogen or a lower alkoxy group, an amino acid or peptide residue provided with a nitrogen protective group conventional in peptide chemistry,

$R_2$ a halogen atom or a lower alkyl group,

$R_3$ and $R_4$, which can be the same or different, each a hydrogen or a halogen atom, whereby, for the case in which $R_2$ and $R_3$ represent a bromine atom and $R_4$ a hydrogen atom,

$R'_1$ does not signify acetyl or benzoyl, characterised in that, in per se known manner, one reacts compounds of the general formula II

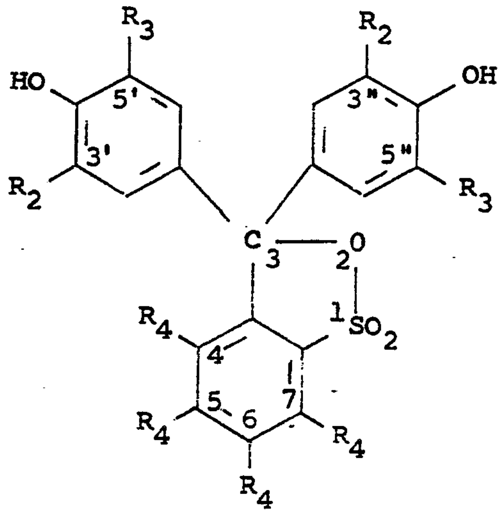

(II),

in which

$R_2$, $R_3$ and $R_4$ have the above-given meanings, with acids of the general formula III

$$HO-R'_1 \qquad (III)$$

in which

$R'_1$ has the above-given meaning, or with suitable reactive derivatives thereof.

**Revendications**

1. Agent de diagnostic pour déceler des leucocytes dans les liquides physiologiques, comprenant un support absorbant imprégné d'un agent chromogène et d'une substance tampon appropriée, caractérisé en ce que l'agent chromogène est choisi parmi les esters de la sulfone phtaléine de la formule générale I

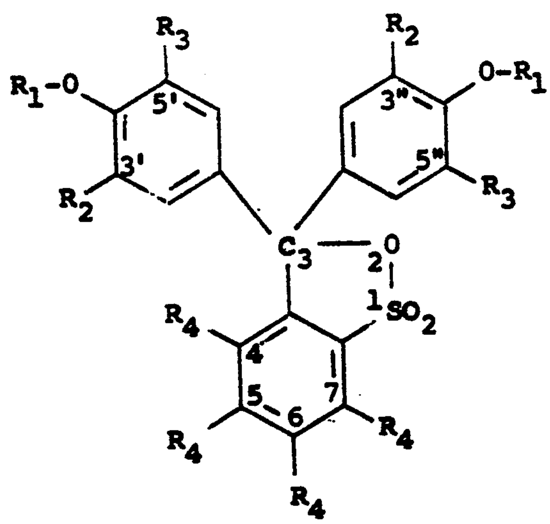

(I),

dans laquelle

$R_1$ est un reste d'acide carbonique éventuellement substitué par un atome d'halogène ou par un

groupe alcoxyle inférieur, ou un reste d'un acide aminé ou d'un peptide, portant un groupe de protection de l'azote habituellement utilisé dans la chimie des peptides,

$R_2$ est un atome d'halogène ou un groupe alkyle inférieur,

$R_3$ et $R_4$, pouvant être identiques ou différents, sont chacun un atome d'hydrogène ou d'halogène.

2. Agent de diagnostic selon la revendication 1, caractérisé en ce que le support renferme des agents auxiliaires additionnels, habituellement utilisés.

3. Agent de diagnostic selon la revendication 2, caractérisé en ce que le support renferme comme agent auxiliaire additionnel un agent mouillant.

4. Utilisation d'esters de sulfone phtaléine de formule générale I

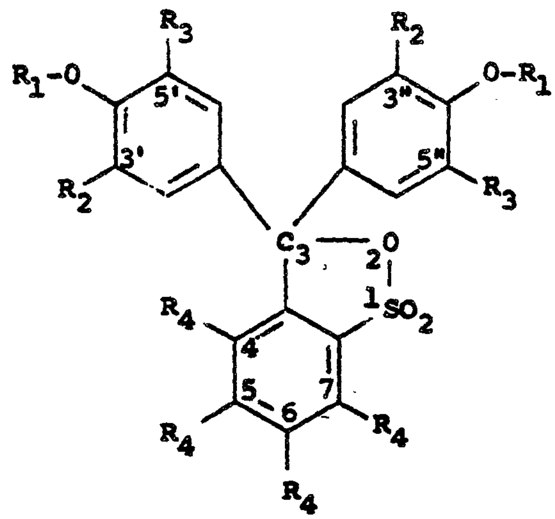

(I),

dans laquelle

$R_1$ est un reste d'acide carbonique éventuellement substitué par un atome d'halogène ou par un groupe alcoxyle inférieur, ou un reste d'un acide aminé ou d'un peptide, portant un groupe de protection de l'azote habituellement utilisé dans la chimie des peptides,

$R_2$ est un atome d'halogène ou un groupe alkyle inférieur,

$R_3$ et $R_4$, pouvant être identiques ou différents, sont chacun un atome d'hydrogène ou d'halogène, pour la préparation d'agents de diagnostic pour déceler des leucocytes.

5. Esters de sulfone phtaléine de formule générale I'

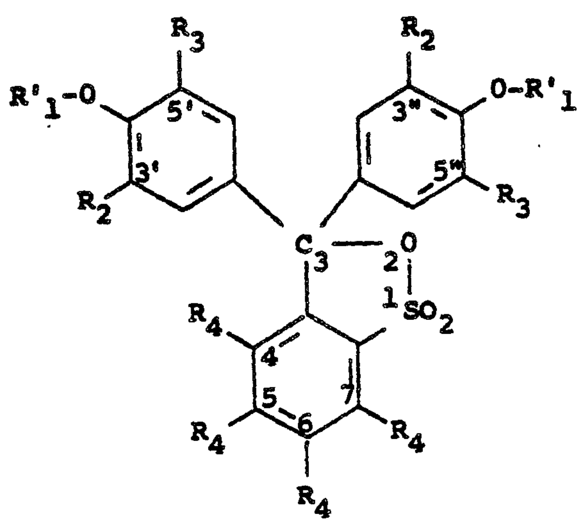

(I'),

dans laquelle

$R_1'$ est un reste d'acide carbonique éventuellement substitué par un atome d'halogène ou par un groupe alcoxyle inférieur, ou un reste d'un acide aminé ou d'un peptide, portant un groupe de protection

de l'azote habituellement utilisé dans la chimie des peptides,

$R_2$ est un atome d'halogène ou un groupe alkyle inférieur,

$R_3$ et $R_4$, pouvant être identiques ou différents, sont chacun un atome d'hydrogène ou d'halogène ; et dans laquelle $R_1'$ dans le cas où $R_2$ et $R_3$ sont chacun un atome de brome et $R_4$ est un atome d'hydrogène, n'est pas un reste acétyle ou benzoyle.

6. Procédé pour la préparation d'esters de sulfone phtaléine de formule générale I'

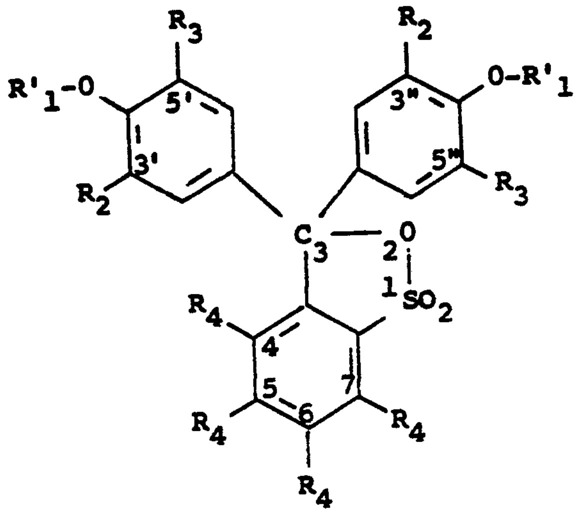

(I'),

dans laquelle

$R_1'$ est un reste d'acide carbonique éventuellement substitué par un atome d'halogène ou par un groupe alcoxyle inférieur, ou un reste d'un acide aminé ou d'un peptide, portant un groupe de protection de l'azote habituellement utilisé dans la chimie des peptides,

$R_2$ est un atome d'halogène ou un groupe alkyle inférieur,

$R_3$ et $R_4$ pouvant être identiques ou différents, sont chacun un atome d'hydrogène ou d'halogène ; et dans laquelle $R_1'$ dans le cas où $R_2$ et $R_3$ sont chacun un atome de brome et $R_4$ est un atome d'hydrogène, n'est pas un reste acétyle ou benzoyle, caractérisé en ce que l'on fait réagir de façon en soi connue des composés de formule générale II

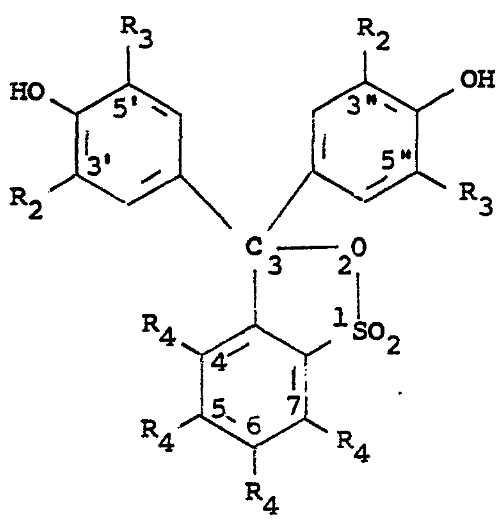

(II),

dans laquelle

$R_2$, $R_3$ et $R_4$ ont la signification ci-dessus indiquée, avec des acides de la formule générale III

$$HO-R_1' \qquad (III),$$

dans laquelle

$R_1'$ a la signification ci-dessus indiquée, ou bien avec des dérivés réactifs appropriés de ces acides.